# EUROPEAN PATENT SPECIFICATION

(11) **EP 1 439 830 B1**
(45) Date of publication and mention of the grant of the patent: **19.10.2005**
(21) Application number: 01274674.9
(22) Date of filing: 02.11.2001
(51) Int. Cl.: A61K 31/192, A61K 47/18

(54) **PHARMACEUTICAL COMPOSITION OF 2-(4-ISOBUTYLPHENYL) PROPIONIC ACID**
PHARMAZEUTISCHE ZUSAMMENSETZUNG MIT 2-(4-ISOBUTYLPHENYL) PROPIONSÄURE
COMPOSITION PHARMACEUTIQUE D'ACIDE PROPIONIQUE 2-(4-ISOBUTYLPHENYLE)

(43) Date of publication of application: 28.07.2004
(73) Proprietor: Cumberland Pharmaceuticals Inc., Nashville, TN 37203 (US)
(72) Inventor: PAVLIV, Leo, Morrisville, NC 27519 (US)
(74) Representative: Grünecker, Kinkeldey, Stockmair & Schwanhäusser Anwaltssozietät
(86) International application number: PCT/US2001/042894
(87) International publication number: WO 2003/039532

(56) References cited:
- WO-A-91/15202
- US-A- 5 693 312
- DATABASE WPI Week 198822 Derwent Publications Ltd., London, GB; AN 1988-152042 XP002203028 & JP 63 093718 A (GREEN CROSS CORP)

## Description

The present invention relates to a pharmaceutical composition for oral or injectable (parenteral) use containing 2-(4-isobutylphenyl) propionic acid and a basic amino acid, and more particularly, where the amino acid is arginine.

### Background of Invention

2-(4-isobutylphenyl) propionic acid, whose International Nonproprietary Name is ibuprofen, is a well-known anti-inflammatory drug having a molecular weight of 206.28 and the following chemical structure: 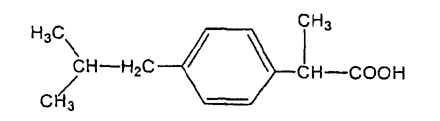 (Merck Index 12th ed., n4925, page 839). Originally patented in the 1960's, ibuprofen is now marketed generically, as well as under the tradenames of Motrin®, Advil®, and Nuprin® for the treatment of pain, inflammation, and fever.

Ibuprofen is readily available as the racemic mixture ((RS)-Ibuprofen) of the two enantiomers, (R)-Ibuprofen and (S)-Ibuprofen. Even though the (S) enantiomer is the biologically active form, most preparations contain the racemic mixture since the (R) enantiomer is converted to the active (S) form in-vivo. For simplicity, hereinafter the term "ibuprofen" will be used to indicate any one of the (R) enantiomer, the (S) enantiomer, or the racemate.

Many amino acids, including arginine, are available as both the D and L forms. For simplicity, hereinafter the term "arginine" will indicate the D or L form of arginine or a mixture of (D)-arginine and (L)-arginine. Arginine has a molecular weight of 174.20.

Although ibuprofen has many advantages over other analgesics such as aspirin and acetaminophen, it is very poorly soluble in water. Thus, certain dosage forms of ibuprofen, especially oral or injectable liquids, have been difficult to develop. Several U.S. patents have addressed this problem.

For example, U.S. Pat. No. 4,309,421 appears to describe watersoluble complexes of ibuprofen and phospholipids suitable for parenteral administration. U.S. Pat. Nos. 4,859,704 and 4,861,797 appear to describe the synthesis of alkali metal salts of ibuprofen for preparing a liquid ibuprofen formulation.

Other U.S. patents appear to address this problem by preparing an ibuprofen salt with a basic amino acid as the active pharmaceutical ingredient and then solubilizing the salt to produce a liquid dosage form.

For example, U.S. Pat. No. 5,200,558 appears to describe enhanced analgesic effects of S (+) ibuprofen as salts of L and D amino acids, including arginine, in various dosage forms, including as an injectable solution. U.S. Pat. No. 4,279,926 appears to describe the use of basic amino acid salts of propionic acids for relieving pain and treating inflammatory conditions. Similarly, U.S. Pat. No. 5,463,117 appears to describe the preparation of salts of ibuprofen with basic amino acids. Finally, U.S. Pat. No. 6,005,005 appears to describe a liquid composition for oral use containing ibuprofen and arginine.
JP 63 093718 appears to describe an injection composition which contains at least one antiinflammatory analgesic selected from mefenamic acid, flufenamic acid, alclofenac and ibuprofen, in the form of a salt with a basic amino acid which is preferably lysine or arginine.

However, the approaches described in the patents discussed above have, among others, the disadvantage of requiring the formation of a salt before solubilization, where the salt must be isolated and tested prior to producing the dosage form. Additionally, the ibuprofen formulations resulting from those processes have at least a 1:1 molar ratio of amino acid to ibuprofen. It is beneficial from both a cost and development point to not have to form a salt and isolate and test it prior to producing the dosage form. It is also beneficial in most cases to minimize the amount of non-active components, including salts, used in therapeutic products in order to minimize potential side effects. Furthermore, for injectable products it is beneficial to produce a liquid dosage form of ibuprofen having a pH similar to that of blood (pH 7.4). Finally, it is beneficial for an injectable and oral product to have similar pharmacokinetics to minimize the need for dosage adjustments.

### Summary of the Invention

The present invention utilizes arginine to solubilize ibuprofen during the manufacture of the pharmaceutical product instead of using a salt form of ibuprofen. Thus, an embodiment of the present invention is a pharmaceutical composition comprising an aqueous solution of arginine and ibuprofen, wherein the molar ratio of arginine to ibuprofen is less than 1:1. Another embodiment of the present invention is a method of making a pharmaceutical composition comprising an aqueous solution of arginine and ibuprofen, wherein the molar ratio of arginine to ibuprofen is less than 1:1. Still other embodiments of the present invention are directed to methods of treating pain, inflammation, fever, and/or other conditions alleviated by ibuprofen comprising administering a pharmaceutical composition comprising an aqueous solution of arginine and ibuprofen, wherein the molar ratio of arginine to ibuprofen is less than 1:1.

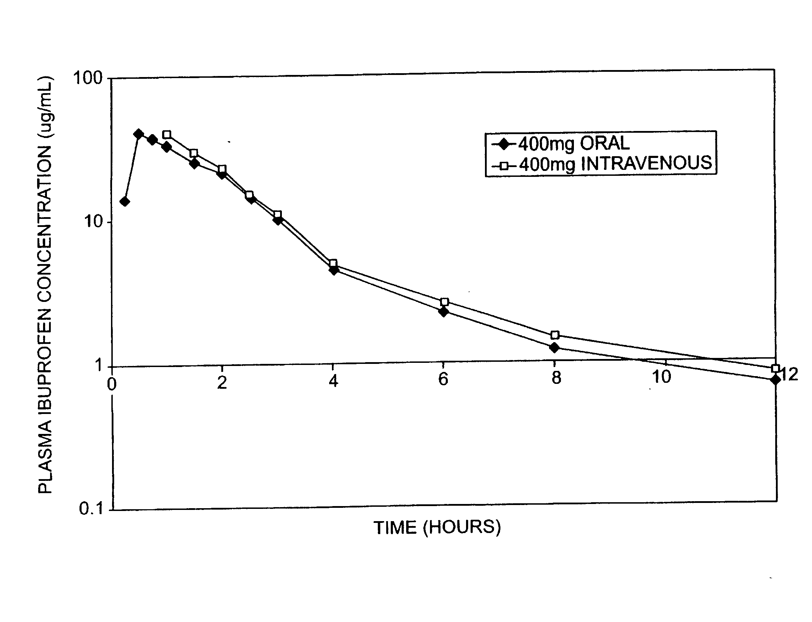

### Brief Description of the Figure

The Figure shows plasma concentration-time curves for 400 mg oral and intravenous ibuprofen.

### Detailed Description of the Invention

The present inventor has discovered that a liquid composition of ibuprofen can be produced by combining ibuprofen with arginine at molar ratios that minimize the amount of arginine necessary to solubilize the ibuprofen. Thus, one embodiment of the present invention is a pharmaceutical composition comprising an aqueous solution of arginine and ibuprofen, wherein the molar ratio of arginine to ibuprofen is less than 1:1. In another embodiment of the invention, the molar ratio of arginine to ibuprofen is from about 0.10:1 to about 0.999:1. In yet other embodiments of the invention, the molar ratio of arginine to ibuprofen is 0.92:1 or 0.60:1 or 0.99:1.

The present inventor has further discovered a method of making a pharmaceutical composition comprising an aqueous solution of arginine and ibuprofen in a molar ratio of less than 1:1, wherein the method comprises the following: adding arginine to water, mixing until the arginine is dissolved to form an arginine solution, adding ibuprofen to the arginine solution, and mixing until the ibuprofen is dissolved to form the aqueous solution of arginine and ibuprofen, optionally adding sufficient water to result in the desired concentration of ibuprofen, and optionally separating any precipitate using standard methods such as filtration or centrifugation. The resulting product is a clear, colorless solution that can readily be passed through a 0.2 micron filter. The pH of the resulting solution can be adjusted using techniques known in the art to achieve a desired pH, for example a pH similar to that of blood. Finally, the resulting solution can be terminally sterilized or lyophilized.

The present inventor has further discovered a method of treating a condition chosen from pain, inflammation, fever, and/or other conditions alleviated by ibuprofen comprising administering to a patient in need thereof an effective amount of a pharmaceutical composition comprising an aqueous solution of arginine and ibuprofen, wherein the molar ratio of arginine to ibuprofen is less than 1:1. Other conditions alleviated by ibuprofen include, but are not limited to, patent ductus arteriosis and certain forms of cancer. The pharmaceutical composition may be administered by injection (intravenous or intramuscular) or orally. Dosages of the pharmaceutical composition range from about 5 to about 100 mg of ibuprofen in the pharmaceutical composition and can be determined by one of ordinary skill in the art. In one embodiment, the dosage is from about 100 to about 800 mg of ibuprofen in the pharmaceutical composition. In a further embodiment, the dosage is about 400 mg of ibuprofen in the pharmaceutical composition. In still another embodiment, the dosage of the pharmaceutical composition is from about 5 to about 10 mg/kg, and in a further embodiment the dosage of the pharmaceutical composition is about 7.5 mg/kg.

The following examples represent specific embodiments of the foregoing discovery, and they are not representative of the entire scope of the invention. The ibuprofen and arginine used in the examples are United States Pharmacopoea grade, but other pharmaceutically acceptable materials can be utilized.

### Example 1

Add 8.2 kg of arginine to approximately 80 liters of water for injection and mix until dissolved. Add 10.0 kg of ibuprofen to the arginine solution and mix until dissolved. Add a sufficient quantity of water to equal 100 liters, resulting in a 100mg/mL solution having a molar ratio of 0.97:1 (arginine:ibuprofen). The product results in a clear, colorless, solution that can readily be passed through a 0.2 micron filter. The pH of the resulting solution is approximately 7.4 and can be adjusted to achieve somewhat lower or higher pH's as desired. The solution can further be terminally sterilized to minimize the likelihood of a non-sterile product.

### Example 2

Lower concentrations of ibuprofen can be prepared by using lesser amounts of arginine and ibuprofen. Add 41 g of arginine to approximately 80 liters of water for injection and mix until dissolved. Add 50 g of ibuprofen to the arginine solution and mix until dissolved. Add a sufficient quantity of water to equal 100 liters, resulting in a 0.5mg/mL solution having a molar ratio of 0.97:1 (arginine:ibuprofen). The product results in a clear, colorless, solution that can readily be passed through a 0.2 micron filter. The pH of the resulting solution can be adjusted to achieve a desirable pH.

### Example 3

Lower concentrations of arginine can be used to prepare the ibuprofen solution. Add 3.8 kg of arginine to approximately 80 liters of water for injection and mix until dissolved. Add 7.5 kg of ibuprofen to the arginine solution and mix until dissolved. Add a sufficient quantity of water to equal 100 liters, resulting in a 75mg/mL solution having a molar ratio of 0.60:1 (arginine:ibuprofen). The product can be passed through a 0.2 micron filter resulting in a clear colorless solution. The pH of the resulting solution can be adjusted to achieve a desirable pH.

### Example 4

Higher concentrations of arginine can be used to prepare the ibuprofen solution. Add 8.43 g of arginine to 80 mL of water for injection and mix until dissolved. Add 10 g of ibuprofen to the arginine solution and mix until dissolved. Add a sufficient quantity of water to equal 100 mL, resulting in a 100mg/mL solution having a molar ratio of 0.99:1 (arginine:ibuprofen). The product results in a clear, colorless, solution that can readily be passed through a 0.2 micron filter. The pH of the resulting solution can be adjusted to achieve a desirable pH.

### Example 5

4.384 kg of arginine were added to approximately 45 liters of water for injection and mixed until dissolved. 5.62 kg of ibuprofen were added to the arginine solution and mixed until dissolved. The pH of the resulting solution was approximately 7.4, but could be adjusted to achieve somewhat lower or higher pH's as desired. A sufficient quantity of water was added to the resulting solution to equal 56.2 liters, resulting in a 100 mg/mL solution having a molar ratio of 0.92:1 (arginine:ibuprofen). The product resulted in a clear, colorless solution that could readily be passed through a 0.2 micron filter. The solution was terminally sterilized to assure that the product was sterilized.

### Example 6

In an attempt to demonstrate similar pharmacokinetics between a 60 minute infusion of intravenous ibuprofen solubilized with arginine as in Example 5 and oral ibuprofen (in the form of Advil® Liqui-Gels®), volunteers received single oral or intravenous doses (200 mg, 400 mg, or 800 mg) of either oral or intravenous ibuprofen product. Blood samples were collected at specified times relative to the start of dosing, and plasma ibuprofen concentrations were measured. The following pharmacokinetic parameters were calculated: Cₘₐₓ(maximum concentration), AUC₀₋₁₂ (area under the curve from initial time to 12 hours), AUC_{0-∞}, (area under the curve from initial time to infinity), Tₘₐₓ (time of maximum concentration), kₑₗ (elimination constant), and t_{½}(half life). Statistical analyses were performed on the plasma concentration data and pharmacokinetic parameters were calculated for the 12 patients on each of the three doses examined.

The plasma concentration-time profiles for both oral and intravenous administration of ibuprofen were observed to be very similar. The concentration-time data for the 400-mg oral and intravenous doses are shown in the Figure to illustrate this result. On the basis of the ibuprofen concentration-time data, the following pharmacokinetic parameters were calculated (Table 1).

**Table 1.**

| **Pharmacokinetic Parameters After Oral and Intravenous Administration of Ibuprofen** | | | | | | |
|---|---|---|---|---|---|---|
| **Parameter** | **Cohort 1: 200 mg** | | **Cohort 2: 400 mg** | | **Cohort 3: 800 mg** | |
| | **Oral** | **Intravenous** | **Oral** | **Intravenous** | **Oral** | **Intravenous** |
| Cₘₐₓ (µg/mL) | 24.7±4.2 | 19.3±3.1 | 42.9±4.9 | 39.2±6.1 | 81.0±18.8 | 72.6±9.6 |
| AUC₀₋₁₂ (µg·hr/mL) | 67.9±16.9 | 63.2±12.5 | 108.0±23.9 | 108.5±29.0 | 211.0±47.6 | 192.2±35.9 |
| AUC_{0-∞} (µg·hr/mL) | 69.9±18.0 | 65.5±14.1 | 110.8±26.8 | 112.3±32.8 | 218.4±55.0 | 197.8±39.9 |
| Tₘₐₓ (hr) | 0.6±0.2 | 1.1±0.2 | 0.6.±0.1 | 1.1±0.2 | 0.9±0.5 | 1.0±0.0 |
| kₑₗ (hr⁻¹) | 0.3±0.0 | 0.3±0.0 | 0.3±0.1 | 0.3±0.1 | 0.3±0.1 | 0.3±0.0 |
| t_{½} (hr) | 2.4±0.2 | 2.3±0.2 | 2.2±0.5 | 2.2±0.5 | 2.3±0.5 | 2.3±0.4 |
| Data shown are mean ± standard deviation. | | | | | | |

The linearity of ibuprofen pharmacokinetics after oral and intravenous administration was analyzed. The results indicated that for both intravenous ibuprofen and oral ibuprofen, AUC₀₋₁₂, AUC_{0-∞}, and Cₘₐₓ increased in an appropriately linear manner with dose.

## Claims

1. A pharmaceutical composition comprising an aqueous solution of arginine and ibuprofen, wherein the molar ratio of arginine to ibuprofen is less then 1:1.

2. The pharmaceutical composition of claim 1, wherein the aqueous solution of arginine and ibuprofen has been terminally sterilized.

3. The pharmaceutical composition of claim 1, wherein the aqueous solution of arginine and ibuprofen has been lyophilized.

4. The pharmaceutical composition of claim 1, wherein the ibuprofen is (RS)-Ibuprofen.

5. The pharmaceutical composition of claim 1, wherein the ibuprofen is (S)-Ibuprofen.

6. The pharmaceutical composition of claim 1, wherein the arginine is L-arginine.

7. The pharmaceutical composition of claim 1, wherein the arginine is D-arginine.

8. The pharmaceutical composition of claim 1, wherein the molar ratio of arginine to ibuprofen is from 0.10:1 to 0.999:1.

9. The pharmaceutical composition of claim 1, wherein the molar ratio of arginine to ibuprofen is 0.92:1.

10. The pharmaceutical composition of claim 8, wherein the molar ratio of arginine to ibuprofen is 0.60:1.

11. The pharmaceutical composition of claim 8, wherein the molar ratio of arginine to ibuprofen is 0.99:1.

12. A method of making a pharmaceutical composition comprising an aqueous solution of arginine and ibuprofen, the method comprising dissolving arginine in water to form an arginine solution and dissolving ibuprofen in the arginine solution to form the aqueous solution of arginine and ibuprofen, wherein the molar ratio of arginine to ibuprofen is less than 1:1.

13. The method of claim 12, wherein the molar ratio of arginine to ibuprofen is from 0.1:1 to 0.999:1.

14. The method of claim 12, wherein the molar ratio of arginine to ibuprofen is 0.92:1.

15. The method of claim 12, wherein the molar ratio of arginine to ibuprofen is 0.60:1.

16. The method of claim 12, wherein the molar ratio of arginine to ibuprofen is 0.99:1.

17. The method of claim 12, further comprising terminally sterilizing the aqueous solution of arginine and ibuprofen.

18. The method of claim 12, further comprising lyophilizing the aqueous solution of arginine and ibuprofen.

19. The method of claim 12, wherein the ibuprofen is (RS)-Ibuprofen.

20. The method of claim 12, wherein the ibuprofen is (S)-Ibuprofen.

21. The method of claim 12, wherein the arginine is L-arginine.

22. The method of claim 12, wherein the arginine is D-arginine.

23. Use of the pharmaceutical composition of claim 1 for the manufacture of a medicament for the treatment of pain, inflammation, fever, and/or another condition alleviated by ibuprofen.

24. The use of claim 23, wherein the molar ratio of arginine to ibuprofen is from 0.10:1 to 0.999:1.

25. The use of claim 23, wherein the molar ratio of arginine to ibuprofen is 0.92:1.

26. The use of claim 23, wherein the molar ratio of arginine to ibuprofen is 0.60:1.

27. The use of claim 23, wherein the molar ratio of arginine to ibuprofen is 0.99:1.

28. The use of claim 23, wherein said use is intravenous.

29. The use of claim 23, wherein said use is intramuscular.

30. The use of claim 23, wherein said use is oral.

31. The use of claim 23, wherein the amount of said composition is from 100 mg to 800 mg of ibuprofen.

32. The use of claim 31, wherein the amount of said composition is 400 mg of ibuprofen.

33. The use of claim 23, wherein the amount of said composition is 7.5 mg/kg of ibuprofen.

34. The use of claim 23, wherein the condition is pain.

35. The use of claim 23, wherein the condition is inflammation.

36. The use of claim 23, wherein the condition is fever.

37. The pharmaceutical composition of claim 1, wherein the molar ratio of arginine to ibuprofen is less than 0.97:1.

38. The pharmaceutical composition of claim 37, wherein said molar ratio of arginine to ibuprofen is from 0.60:1 to 0.97:1.

39. The pharmaceutical composition of claim 37, wherein said molar ratio is from 0.92:1 to 0.97:1.

40. The method of claim 12, wherein the molar ratio of arginine to ibuprofen is less than 0.97:1.

41. The method of claim 40, wherein the molar of arginine to ibuprofen is from 0.60:1 to 0.97:1.

42. The method of claim 40, wherein the molar ratio of arginine to ibuprofen is from 0.92:1 to 0.97:1.

43. The use of claim 23, wherein the molar ratio of arginine to ibuprofen is less than 0.97:1.

44. The use of claim 43, wherein the molar ratio of arginine to ibuprofen is from 0.60:1 to 0.97:1.

45. The use of claim 43, wherein the molar ratio of arginine to ibuprofen is from 0.92:1 to 0.97:1.

46. The pharmaceutical composition of claim 1, wherein the pH of the solution can be adjusted to achieve a desired pH similar to that of blood.

47. The method of claim 12, wherein the pH of the solution can be adjusted to achieve a desired pH similar to that of blood.

48. The use of claim 23, wherein the pH of the solution can be adjusted to achieve a desired pH similar to that of blood.

## Patentansprüche

1. Pharmazeutische Zusammensetzung, umfassend eine wässrige Lösung von Arginin und Ibuprofen, worin das molare Verhältnis von Arginin zu Ibuprofen weniger als 1 : 1 beträgt.

2. Pharmazeutische Zusammensetzung nach Anspruch 1, worin die wässrige Lösung aus Arginin und Ibuprofen in der Endverpackung sterilisiert wird (terminally sterilized).

3. Pharmazeutische Zusammensetzung nach Anspruch 1, worin die wässrige Lösung aus Arginin und Ibuprofen lyophilisiert wurde.

4. Pharmazeutische Zusammensetzung nach Anspruch 1, worin das Ibuprofen (RS)-Ibuprofen ist.

5. Pharmazeutische Zusammensetzung nach Anspruch 1, worin das Ibuprofen (S)-Ibuprofen ist.

6. Pharmazeutische Zusammensetzung nach Anspruch 1, worin das Arginin L-Arginin ist.

7. Pharmazeutische Zusammensetzung nach Anspruch 1, worin das Arginin D-Arginin ist.

8. Pharmazeutische Zusammensetzung nach Anspruch 1, worin das molare Verhältnis von Arginin zu Ibuprofen von 0,10 : 1 bis 0,999 : 1 beträgt.

9. Pharmazeutische Zusammensetzung nach Anspruch 1, worin das molare Verhältnis von Arginin zu Iboprofen 0,92 : 1 beträgtt.

10. Pharmazeutische Zusammensetzung nach Anspruch 8, worin das molare Verhältnis von Arginin zu Ibuprofen 0,60 : 1 beträgt.

11. Pharmazeutische Zusammensetzung nach Anspruch 8, worin das molare Verhältnis von Arginin zu Ibuprofen 0,99 : 1 beträgt.

12. Verfahren zur Herstellung einer pharmazeutischen Zusammensetzung, umfassend eine wässrige Lösung aus Arginin und Ibuprofen, wobei das Verfahren das Auflösen von Arginin in Wasser umfasst, um eine Argininlösung herzustellen und das Auflösen von Ibuprofen in der Argininlösung, um eine wässrige Lösung aus Arginin und Ibuprofen herzustellen, worin das molare Verhältnis von Arginin zu Ibuprofen weniger als 1 : 1 beträgt.

13. Verfahren nach Anspruch 12, worin das molare Verhältnis von Arginin zu Ibuprofen von 0,1 : 1 bis 0,999 : 1 beträgt.

14. Verfahren nach Anspruch 12, worin das molare Verhältnis von Arginin zu Ibuprofen 0, 92 : 1 beträgt.

15. Verfahren nach Anspruch 12, worin das molare Verhältnis von Arginin zu Ibuprofen 0,60 : 1 beträgt.

16. Verfahren nach Anspruch 12, worin das molare Verhältnis von Arginin zu Ibuprofen 0,99 : 1 beträgt.

17. Verfahren nach Anspruch 12, weiterhin umfassend das Sterilisieren der wässrigen Lösung von Arginin und Ibuprofen in der Endverpackung.

18. Verfahren nach Anspruch 12, weiterhin umfassend das Lyophilisieren der wässrigen Lösung aus Arginin und Ibuprofen.

19. Verfahren nach Anspruch 12, worin das Ibuprofen (RS)-Ibuprofen ist.

20. Verfahren nach Anspruch 12, worin das Ibuprofen (S)-Ibuprofen ist.

21. Verfahren nach Anspruch 12, worin das Arginin L-Arginin ist.

22. Verfahren nach Anspruch 12, worin das Arginin D-Arginin ist.

23. Verwendung der pharmazeutischen Zusammensetzung nach Anspruch 1 für die Herstellung eines Arzneimittels zur Behandlung von Schmerzen, Entzündung, Fieber und/oder weiteren Zuständen, die durch Ibuprofen gelindert werden.

24. Verwendung nach Anspruch 23, worin das molare Verhältnis von Arginin zu Ibuprofen von 0,10 : 1 bis 0,999 : 1 beträgt.

25. Verwendung nach Anspruch 23, worin das molare Verhältnis von Arginin zu Ibuprofen 0,92 : 1 beträgt.

26. Verwendung nach Anspruch 23, worin das molare Verhältnis von Arginin zu Ibuprofen 0,60 : 1 beträgt.

27. Verwendung nach Anspruch 23, worin das molare Verhältnis von Arginin zu lbuprofen 0,99 : 1 beträgt.

28. Verwendung nach Anspruch 23, worin die Verwendung intravenös ist.

29. Verwendung nach Anspruch 23, worin die Verwendung intramuskulär ist.

30. Verwendung nach Anspruch 23, worin die Verwendung oral ist.

31. Verwendung nach Anspruch 23, worin die Menge der Zusammensetzung von 100 mg bis 800 mg Ibuprofen beträgt.

32. Verwendung nach Anspruch 31, worin die Menge der Zusammensetzung 400 mg Ibuprofen beträgt.

33. Verwendung nach Anspruch 23, worin die Menge der Zusammensetzung 7,5 mg/kg Ibuprofen beträgt.

34. Verwendung nach Anspruch 23, worin der Zustand Schmerz ist.

35. Verwendung nach Anspruch 23, worin der Zustand Entzündung ist.

36. Verwendung nach Anspruch 23, worin der Zustand Fieber ist.

37. Pharmazeutische Zusammensetzung nach Anspruch 1, worin das molare Verhältnis von Arginin zu Ibuprofen weniger als 0,97 : 1 beträgt.

38. Pharmazeutische Zusammensetzung nach Anspruch 37, worin das molare Verhältnis von Arginin zu Ibuprofen von 0,60 : 1 bis 0,97 : 1 beträgt.

39. Pharmazeutische Zusammensetzung nach Anspruch 37, worin das molare Verhältnis von 0,92 : 1 bis 0,97 : 1 beträgt.

40. Verfahren nach Anspruch 12, worin das molare Verhältnis von Arginin zu Ibuprofen weniger als 0,97 : 1 beträgt.

41. Verfahren nach Anspruch 40, worin das molare Verhältnis von Arginin zu Ibuprofen von 0,60 : 1 bis 0,97 : 1 beträgt.

42. Verfahren nach Anspruch 40, worin das molare Verhältnis von Arginin zu Ibuprofen 0,92 : 1 bis 0,97 : 1 beträgt.

43. Verwendung nach Anspruch 23, worin das molare Verhältnis von Arginin zu Ibuprofen weniger als 0,97 : 1 beträgt.

44. Verwendung nach Anspruch 43, worin das molare Verhältnis von Arginin zu Ibuprofen 0,60 : 1 bis 0,97 : 1 beträgt.

45. Verwendung nach Anspruch 43, worin das molare Verhältnis von Arginin zu Ibuprofen von 0,92 : 1 bis 0,97 : 1 beträgt.

46. Pharmazeutische Zusammensetzung nach Anspruch 1, worin der pH der Lösung eingestellt werden kann, um einen gewünschten pH-Wert ähnlich dem von Blut zu erhalten.

47. Verfahren nach Anspruch 12, worin der pH der Lösung eingestellt werden kann, um einen gewünschten pH-Wert ähnlich dem von Blut zu erhalten.

48. Verwendung nach Anspruch 23, worin der pH der Lösung eingestellt werden kann, um einen gewünschten pH-Wert ähnlich dem von Blut zu erhalten.

## Revendications

1. Composition pharmaceutique comprenant une solution aqueuse d'arginine et d'ibuprofène, dans laquelle le rapport molaire entre l'arginine et l'ibuprofène est inférieur à 1:1.

2. Composition pharmaceutique selon la revendication 1, dans laquelle la solution aqueuse d'arginine et d'ibuprofène est au final stérilisée.

3. Composition pharmaceutique selon la revendication 1, dans laquelle la solution aqueuse d'arginine et d'ibuprofène est lyophilisée.

4. Composition pharmaceutique selon la revendication 1, dans laquelle l'ibuprofène est le (RS)-ibuprofène.

5. Composition pharmaceutique selon la revendication 1, dans laquelle l'ibuprofène est le (S)-ibuprofène.

6. Composition pharmaceutique selon la revendication 1, dans laquelle l'arginine est la L-arginine.

7. Composition pharmaceutique selon la revendication 1, dans laquelle l'arginine est la D-arginine.

8. Composition pharmaceutique selon la revendication 1, dans laquelle le rapport molaire entre l'arginine et l'ibuprofène est de 0,10:1 à 0,999:1.

9. Composition pharmaceutique selon la revendication 1, dans laquelle le rapport molaire entre l'arginine et l'ibuprofène est 0,92:1.

10. Composition pharmaceutique selon la revendication 8, dans laquelle le rapport molaire entre l'arginine et l'ibuprofène est 0,60:1.

11. Composition pharmaceutique selon la revendication 8, dans laquelle le rapport molaire entre l'arginine et l'ibuprofène est 0,99:1.

12. Procédé de fabrication d'une composition pharmaceutique comprenant une solution aqueuse d'arginine et d'ibuprofène, le procédé comprenant la dissolution de l'arginine dans l'eau pour former une solution d'arginine et la dissolution de l'ibuprofène dans la solution d'arginine pour former la solution aqueuse d'arginine et d'ibuprofène, dans lequel le rapport molaire entre l'arginine et l'ibuprofène est inférieur à 1:1.

13. Procédé selon la revendication 12, dans lequel le rapport molaire entre l'arginine et l'ibuprofène est de 0,1:1 à 0,999:1.

14. Procédé selon la revendication 12, dans lequel le rapport molaire entre l'arginine et l'ibuprofène est 0,92:1.

15. Procédé selon la revendication 12, dans lequel le rapport molaire entre l'arginine et l'ibuprofène est 0,60:1.

16. Procédé selon la revendication 12, dans lequel le rapport molaire entre l'arginine et l'ibuprofène est 0,99:1.

17. Procédé selon la revendication 12, comprenant en outre la stérilisation finale de la solution aqueuse d'arginine et d'ibuprofène.

18. Procédé selon la revendication 12, comprenant en outre la lyophilisation de la solution aqueuse d'arginine et d'ibuprofène.

19. Procédé selon la revendication 12, dans lequel l'ibuprofène est le (RS)-ibuprofène.

20. Procédé selon la revendication 12, dans lequel l'ibuprofène est le (S)-ibuprofène.

21. Procédé selon la revendication 12, dans lequel l'arginine est la L-arginine.

22. Procédé selon la revendication 12, dans lequel l'arginine est la D-arginine.

23. Utilisation de la composition pharmaceutique selon la revendication 1 pour la fabrication d'un médicament destiné au traitement de la douleur, de l'inflammation, de la fièvre et/ou d'un autre état pathologique soulagé par l'ibuprofène.

24. Utilisation selon la revendication 23, dans laquelle le rapport molaire entre l'arginine et l'ibuprofène est de 0,10:1 à 0,999:1.

25. Utilisation selon la revendication 23, dans laquelle le rapport molaire entre l'arginine et l'ibuprofène est 0,92:1.

26. Utilisation selon la revendication 23, dans laquelle le rapport molaire entre l'arginine et l'ibuprofène est 0,60:1.

27. Utilisation selon la revendication 23, dans laquelle le rapport molaire entre l'arginine et l'ibuprofène est 0,99:1.

28. Utilisation selon la revendication 23, dans laquelle ladite utilisation est intraveineuse.

29. Utilisation selon la revendication 23, dans laquelle ladite utilisation est intramusculaire.

30. Utilisation selon la revendication 23, dans laquelle ladite utilisation est orale.

31. Utilisation selon la revendication 23, dans laquelle la quantité de ladite composition est de 100 mg à 800 mg d'ibuprofène.

32. Utilisation selon la revendication 31, dans laquelle la quantité de ladite composition est 400 mg d'ibuprofène.

33. Utilisation selon la revendication 23, dans laquelle la quantité de ladite composition est 7,5 mg/kg d'ibuprofène.

34. Utilisation selon la revendication 23, dans laquelle l'état pathologique est la douleur.

35. Utilisation selon la revendication 23, dans laquelle l'état pathologique est l'inflammation.

36. Utilisation selon la revendication 23, dans laquelle l'état pathologique est la fièvre.

37. Composition pharmaceutique selon la revendication 1, dans laquelle le rapport molaire entre l'arginine et l'ibuprofène est inférieur à 0,97:1.

38. Composition pharmaceutique selon la revendication 37, dans laquelle ledit rapport molaire entre l'arginine et l'ibuprofène est de 0,60:1 à 0,97:1.

39. Composition pharmaceutique selon la revendication 37, dans laquelle ledit rapport molaire est de 0,92:1 à 0,97:1.

40. Procédé selon la revendication 12, dans lequel le rapport molaire entre l'arginine et l'ibuprofène est inférieur à 0,97:1.

41. Procédé selon la revendication 40, dans lequel le rapport molaire entre l'arginine et l'ibuprofène est de 0,60:1 à 0,97:1.

42. Procédé selon la revendication 40, dans lequel le rapport molaire entre l'arginine et l'ibuprofène est de 0,92:1 à 0,97:1.

43. Utilisation selon la revendication 23, dans laquelle le rapport molaire entre l'arginine et l'ibuprofène est inférieur à 0,97:1.

44. Utilisation selon la revendication 43, dans laquelle le rapport molaire entre l'arginine et l'ibuprofène est de 0,60:1 à 0,97:1.

45. Utilisation selon la revendication 43, dans laquelle le rapport molaire entre l'arginine et l'ibuprofène est de 0,92:1 à 0,97:1.

46. Composition pharmaceutique selon la revendication 1, dans laquelle le pH de la solution peut être ajusté pour atteindre un pH souhaité similaire à celui du sang.

47. Procédé selon la revendication 12, dans lequel le pH de la solution peut être ajusté pour atteindre un pH souhaité similaire à celui du sang.

48. Utilisation selon la revendication 23, dans laquelle le pH de la solution peut être ajusté pour atteindre un pH souhaité similaire à celui du sang.
